# EUROPÄISCHE PATENTSCHRIFT

(11) **EP 0 930 916 B1**
(45) Veröffentlichungstag und Bekanntmachung des Hinweises auf die Patenterteilung: **12.09.2001**
(21) Anmeldenummer: 97944727.3
(22) Anmeldetag: 22.09.1997
(51) Int. Cl.: A61N 5/06, A61B 5/00

(54) **VORRICHTUNG ZUR PRÜFUNG UND/ODER JUSTIERUNG EINES PDD- ODER PDT-SYSTEMS UND/ODER ZUR SCHULUNG AN EINEM DERARTIGEN SYSTEM**
TESTING AND/OR SETTING DEVICE FOR A PHOTODYNAMIC DIAGNOSIS OR THERAPY SYSTEM, OR FOR TRAINING ON SUCH A SYSTEM
DISPOSITIF DE CONTROLE ET/OU REGLAGE D'UN SYSTEME DE DIAGNOSTIC OU TRAITEMENT PHOTODYNAMIQUE, OU DE FORMATION SUR UN TEL SYSTEME

(30) Priorität: 20.09.1996 DE 19638809
(43) Veröffentlichungstag der Anmeldung: 28.07.1999
(73) Patentinhaber: Karl Storz GmbH & Co. KG, 78532 Tuttlingen (DE)
(72) Erfinder: IRION, Klaus, D-78576 Liptingen (DE); EHRHARDT, Andre, D-78532 Tuttlingen (DE); REBHOLZ, Clemens, D-88690 Uhldingen-Mühlhofen (DE); STROBL, Karl-Heinz, Fiskdale, MA 01518 (US)
(74) Vertreter: Münich, Wilhelm, Dr.
(86) Internationale Anmeldenummer: DE9702138
(87) Internationale Veröffentlichungsnummer: WO9811945

(56) Entgegenhaltungen:
- WO-A-87/07131
- WO-A-96/36273
- US-A- 5 068 515

## Beschreibung

### Technisches Gebiet

Die Erfindung bezieht sich auf eine Vorrichtung zur Prüfung und/oder Justierung eines PDD- oder PDT-Systems und/oder zur Schulung an einem derartigen System.

### Stand der Technik

Systeme zur photodynamischen Diagnose (PDD) und/oder photodynamischen Therapie (PDT) werden in der Medizin vielfältig eingesetzt. Nur exemplarisch werden ophthalmologische, endoskopische oder neurochirurgische Anwendungen genannt:

Mit der photodynamischen Diagnose und Therapie werden beispielsweise maligne, aber auch benigne Gewebeentartungen erkannt bzw. behandelt. Hierzu werden Photosensibilisatoren verabreicht, die sich spezifisch in dem zu erkennenden Gewebe anreichern und bei Beleuchtung mit Anregungslicht fluoreszieren (photodynamische Diagnose) bzw. bei hoher Dosis des Photosensibilisators und großer Beleuchtungsstärke zu einem phototoxischen Effekt führen, durch den das entartete Gewebe zerstört wird (photodynamische Terapie). Zur photodynamischen Diagnose können auch körpereigene Fluorezsenzstoffe herangezogen werden. Es handelt sich dann um sogenannte Autofluoreszine.

Dabei ist der Wellenlängenbereich des Anregungslichts kurzwelliger als der Wellenlängenbereich, in dem Fluoreszenzlicht auftritt.

Bei den bekannten PDD- bzw. PDT-Systemen besteht das Problem, daß die gewebecharakterisierende Fluoreszenz, die durch den verabreichten Photosensibilisator und die Beaufschlagung mit Anregungslicht hervorgerufen wird, nur "in-vivo" erkannt werden kann. Dies bedeutet, daß die bekannten PDD- bzw. PDT-Systeme derzeit nur unter "in-vivo"-Bedingungen voll umfänglich geprüft bzw. verifiziert werden können.

Darüberhinaus kann auch die Schulung bzw. Ausbildung an PDD- bzw. PDT-Systemen derzeit nur "in-vivo" erfolgen. Damit muß entweder eine vergleichsweise große Patienten-Belastung oder eine unzureichende Schulung mit der Folge, daß beispielsweise maligne Gewebe bzw. Karzinome nicht erkannt werden, in Kauf genommen werden.

WO-A-87-7131 betrifft ein Beleuchtungssystem womit mit Fluoreszenzmittel angereichertes menschlisches Gewebe beleuchtet wird US-A-5.068.501 betrifft ein optisches System womit die Intensität in einem Laserstrahl homogenisiert werden kann.

Der Erfindung liegt die Aufgabe zugrunde, eine Vorrichtung anzugeben, mit der PDD- bzw. PDT-Systeme "ex-vivo" geprüft und/oder eine Schulung an derartigen Systemen durchgeführt werden kann.

Eine erfindungsgemäße Lösung dieser Aufgabe ist im Patentanspruch 1 angegeben. Weiterbildungen der Erfindung sind Gegenstand der Ansprüche 2 f..

### Darstellung der Erfindung

Erfindungsgemäß wird eine Vorrichtung zur Prüfung und/ oder Justierung eines PDD- oder PDT-Systems und/oder zur Schulung an einem derartigen System geschaffen, die ein Target aufweist, das das Licht des Beleuchtungs-systems des PDD- oder PDT-Systems reflektiert und das wenigstens eine Lichtquelle aufweist, die Licht im Wellenlängenbereich des Fluoreszenzspektrums des jeweiligen Photosensibilisators oder Fluoreszenzstoffs abstrahlt.

Die erfindungsgemäße Vorrichtung ermöglicht damit sowohl die Prüfung des Beleuchtungssystems als auch der bildgebenden Einheit des PDD- bzw. PDT-Systems. Insbesondere ist es möglich, daß der Hersteller eines derartigen Systems vor Auslieferung des Systems an einen Kunden die Funktionalität des Systems einschließlich der Fluoreszenzdetektion überprüft. Dabei kann das System auch zur Justierung der einzelnen Bauteile und insbesondere zur Überprüfung der Justierung eines eventuell vorgesehenen Filtersystems eingesetzt werden.

Vor allem aber ermöglicht es die erfindungsgemäße Vorrichtung, Ärzte in der Verwendung derartiger Systeme zu schulen bzw. es gibt Ärzten die Möglichkeit, bei einem Negativ-Ergebnis der PDD-Untersuchung sich vom einwandfreien Zustand des verwendeten PDD-Systems zu überzeugen.

Dabei sind im Rahmen des erfindungsgemäßen Grundgedankens die verschiedensten Ausgestaltungen möglich:

So ist es möglich, daß das Target mehrere Lichtquellen aufweist, so daß das Licht, das im Wellenlängenbereich des Fluoreszenzspektrums des jeweiligen Photosensibilisators abgestrahlt wird, von einer "größeren Fläche" kommt. Insbesondere um der Person, die an einem PDD-bzw. PDT-System geschult werden soll, bestimmte Muster anzubieten, die diese Person erkennen muß, ist es möglich, die Lichtquellen selektiv derart anzusteuern, daß sie Licht in Form vorgebbarer Muster emittieren. Zusätzlich oder alternativ kann die Größe der Lichtemittierenden Fläche der Lichtquellen variiert werden.

Weiterhin können die Lichtquellen verschieden große Abstrahlflächen aufweisen, die unter anderem dazu benutzt werden können, eine von der zu untersuchenden Person gerade noch erkennbare Ausdehnung von Fluoreszenzbereichen zu ermitteln. Somit können die Auflösungsgrenzen ermittelt werden.

Zur Justierung des Systems ist es ferner bevorzugt, wenn das Target wenigstens einen Sensor aufweist, der die Stärke des Beleuchungslichtes erfaßt, und dessen Ausgangssignal an einer Steuereinheit anliegt, die die Stärke des von der oder den Lichtquellen emittierten Lichtes regelt. Um einer Person, die an einem PDD- oder PDT-System geschult werden soll, eine "Vorstellung" von der Wirkungsweise eines-derartigen Systems zu geben, ist es weiterhin von Vorteil, wenn der wenigstens eine Sensor die Stärke des Beleuchungslichtes nur in dem Wellenlängenbereich erfaßt, durch den der jeweils verwendete Photosensibilisator angeregt wird. Dabei ist es ferner möglich, daß die Steuereinheit die Lichtquelle(n) des Targets derart steuert, daß mit zunehmender Zeitdauer diese Lichtquellen weniger hell strahlen und so das Abklingen der Wirksamkeit des Fluoreszenzmarkers simuliert wird.

Bei einer Reihe von PDD- bzw. PDT-Systemen strahlt die Beleuchtungseinheit nicht nur Licht in dem Wellenbereich, durch den der Photosensibilisator angeregt wird, ab, sondern auch in anderen Wellenlängenbereichen ab, so daß das bestrahlte Gewebe zusätzlich beleuchtet wird. Alternativ kann eine weitere Lichtquelle oder weitere Lichtquellen vorgesehen sein, die zusätzlich zu dem Anregungslicht Beleuchtungslicht abstrahlen. Bei derartigen PDD- bzw. PDT-Systemen ist es bevorzugt, wenn ein Filtersystem vorgesehen ist, das das an dem Gewebebereich bzw. dem Target direkt reflektierte Anregungs-Licht abblockt, so daß es für die Untersuchungsperson leichter wird, die Fluoreszenzstrahlung vor dem "Hintergrund" des eingestrahlten Lichts zu erkennen.

Dieses Filtersystem kann insbesondere entsprechend einem Vorschlag der Karl Storz GmbH & Co. derart ausgebildet sein, daß das Filtersystem den Reintransmissionsgrad des Beleuchtungssystems dem Fluoreszenzanregungsspektrum des jeweiligen Photosensibilisators und den Reintransmissionsgrad der bildgebenden Einheit dem Fluoreszenzspektrum des jeweiligen Photosensibilisators derart anpaßt, daß sich die beiden Reintransmissionsgrade nur in einem vergleichsweise kleinen Wellenbereich nennenswert überlappen. Dieser kleine Wellenlängenbereich kann insbesondere so gewählt werden, daß das in ihm reflektierte Licht eine ausreichende "Hintergrundbeleuchtung" für das Fluoreszenzlicht ergibt, so daß die Untersuchungsperson den beleuchteten Gewebebereich unabhängig von dem Fluoreszenzlicht "erkennen" kann.

Die visuelle Erkennbarkeit der Fluoreszenzstrahlung kann weiter dadurch verbessert werden, daß die Intensität und/oder die Wellenlängen-Erstreckung des lediglich zur Beleuchtung dienenden Lichts einstellbar ist. Diese Einstellung kann beispielsweise dadurch erfolgen, daß eine Wellenlängen-durchstimmbare Lichtquelle verwendet wird oder einer Lichtquelle mit festem Emissionsspektrum ein durchstimmbares Filtersystem vorgeschaltet wird.

Die Transmissionskurve dieses Filtersystems kann ebenso wie die Transmissionskurve des Filtersystems der bildgebenden Einheit einstellbar sein.

Die Verwendung von Filtern in der bildgebenden Einheit kann darüberhinaus wie folgt genutzt werden:

Insbesondere wenn zusätzlich zum Anregungslicht Licht eines weiteren Wellenlängenbereichs eingekoppelt wird, das als Hintergrundbeleuchtung für den Bereich dient, aus dem Fluoreszenzlicht emittiert wird, kann durch geeignete Wahl der Filter bzw. Einstellung der Filter-Wellenlängen eine kontrastreichere Farbdifferenzierung erhalten werden.

Gemäß diesem Ausführungsbeispiel kann die Vorrichtung damit auch zur Bestimmung der optischen Farbdifferenzierung zwischen Fluoreszenzeffekt und Hintergrundlicht herangezogen werden. Darüberhinaus können Benutzer eines PDD-Systems die erfindungsgemäße Vorrichtung dazu nutzen, das System auf den jeweils subjektiv stärksten Farbkontrast einzustellen.

Weiterhin ist es möglich, in den Lichtweg der bildübertragenden Einheit umschaltbare Filter einzubringen. Werden diese Filter mit hoher Frequenz umgeschaltet, so ist es möglich, den Kontrast zwischen Fluoreszenzstrahlung und am Gewebe bzw. Target reflektierter Hintergrundbeleuchtung weiter zu erhöhen. Wenn die bildgebende Einheit eine Videokamera aufweist, ist es bevorzugt, wenn die Umschaltung der Filter mit einer Frequenz erfolgt, die ein 1/n-faches (n=1..200) der Videofrequenz ist.

In jedem Falle ist es von Vorteil, wenn das Target das Licht des Beleuchtungssystems zumindest in dem Teil des Spektrums, der zur Anregung des Photosensibilisators dient, angenähert wie das Körpergewebe reflektiert, auf das das Licht des Beleuchtungssystems gerichtet werden soll. Hierzu kann das Target eine Oberflächenstruktur aufweisen, die insbesondere bezüglich Farbe und Rauheit in etwa der des zu simulierenden Gewebes entspricht. Damit ist es möglich, PDD- bzw. PDT-Systeme realistisch zu prüfen und insbesondere den Kontrast des erhaltenen Bildes durch Justieren von Filtersystemen bzw. Emissionsspektren geeignet einzustellen.

Die Anpassung des Targets an das jeweils zu analysierende Körpergewebe kann beispielsweise dadurch erfolgen, daß das Target in Einfalls-Richtung des Beleuchtungslichtes vor der Fläche, in der sich die Lichtquelle(n) befindet(n), ein wellenlängen-selektiv reflektierende Fläche oder ein Filterelement aufweist.

Die erfindungsgemäße Vorrichtung eignet sich zur Prüfung von bzw. zur Schulung an beliebigen PDD- bzw. PDT-Systemen. Besonders vorteilhaft ist der Einsatz von erfindungsgemäßen Vorrichtungen jedoch in Verbindung mit PDD- bzw. PDT-Systemen, bei denen zusätzlich auch Licht zur Hintergrundbeleuchtung bzw. zur Beleuchtung des fluoreszierenden Bereichs eingekoppelt wird. Derartige Systeme können insbesondere ein Endoskop oder ein Operationsmikroskop aufweisen, in das das Beleuchtungs- sowie das Anregungslicht eingekoppelt wird, und dessen Abbildungssystem Teil der bildgebenden Einheit ist. Der Einsatzbereich derartiger PDD- bzw. PDT-Systeme deckt nahezu den gesamten Bereich der Medizin ab und legt insbesondere in endoskopischen Untersuchungs- bzw. Operationsverfahren, ophthalmologischen Behandlungen oder in der Neurochirurgie.

Die hierfür benötigten Vorrichtungen werden bevorzugt mit einer erfindungsgemäßen Vorrichtung getestet, die einen Hohlraum aufweist, die zur Prüfung und/oder zur Schulung das distale Ende des jeweils verwendeten Endoskops bzw. das Mikroskop-Objektiv eingesetzt wird. Damit kann das Endoskop bzw. das Mikroskop unter realistischen Bedingungen und insbesondere unter Lichtverhältnissen getestet werden, wie sie auch im Körperinneren vorliegen.

Weiterhin ist es bevorzugt, wenn in den Hohlraum eine Sterilfolie eingesetzt wird, die das eingesetzte Teil vor Kontamination schützt. Damit ist es möglich, Endoskope oder Operationsmikroskope auch während einer Operation zu testen, ohne daß die Sterilität dieser Teile beeinträchtigt würde.

Die Realitätsnähe des mit der erfindungsgemäßen Vorrichtung durchgeführten Tests bzw. der Schulung wird weiter verbessert, wenn das Target eine Krümmung entsprechend der Objektfeldkrümmung der bildgebenden Einheit aufweist. Diese ist in der Regel der Krümmung des Organs angepaßt, in die das Endoskop eingesetzt bzw. das mit dem Operationsmikroskop betrachtet wird.

Die Lichtquelle bzw. die Lichtquellen des Targets können in den verschiedensten Arten ausgebildet sein: So können Leuchtdioden oder Miniaturlämpchen vorgesehen sein. Ferner ist es möglich, daß die Lichtquellen nicht in der Targetoberfläche, sondern beabstandet von dieser angeordnet ist. Das Licht der Lichtquelle wird über Lichtleitfasern in die Targetoberfläche geleitet, in der die Austrittsflächen der Lichtleitflächen der Lichtleitfasern liegen. Weiterhin ist es möglich, einen Monitorbildschirm oder dergleichen in der Targetoberfläche anzuordnen.

Bei allen diesen Lösungen ist es jedoch von Vorteil, wenn das Emissionsspektrum der Lichtquellen dem Fluoreszenzspektrum des jeweiligen Photosensibilisators angepaßt ist. Diese Anpassung kann beispielsweise durch ein in den Lichtweg der bildgebenden Einheit eingebrachtes Filtersystem erfolgen, das das Licht der Lichtquellen so filtert, daß das Bild des Targets am proximalen Ende ein Spektrum aufweist, das dem Fluoreszenzspektrum des jeweiligen Photosensibilisators bzw. des körpereigenen Floureszenzstoffes zumindest angenähert gleicht.

Zur Steuerung der verschiedenen Funktionen einschließlich der Regelung der Stärke des von der oder den Lichtquellen des Targets emittierten Lichtes kann eine Steuer- und Auswerteeinheit vorgesehen sein. Diese Steuer- und Auswerteeinheit, die beispielsweise ein handelsüblicher PC mit entsprechenden Zusatzkarten sein kann, kann die einzelnen Funktionen nach einem vorgebbaren Programmablauf steuern und insbesondere Schulungsprogramme ausführen. Darüberhinaus können an der Steuer- und Auswerteeinheit die Signale der verschiedenen vorgesehenen Sensoren und insbesondere eines Bildsensors, wie eines Videochips der bildgebenden Einheit angelegt sein.

### Kurze Beschreibung der Zeichnung

Die Erfindung wird nachstehend ohne Beschränkung des allgemeinen Erfindungsgedankens anhand von Ausführungsbeispielen unter Bezugnahme auf die Zeichnung exemplarisch beschrieben, auf die im übrigen bezüglich der Offenbarung aller im Text nicht näher erläuterten erfindungsgemäßen Einzelheiten ausdrücklich verwiesen wird. Es zeigen:
- Figur 1: eine erfindungsgemäße Vorrichtung, und
- Figur 2: die Abhängigkeit der Leistungsdichte der abstrahlenden Lichtquellen von der Bestrahlungsstärke des Anregungslichtes.

### Darstellung eines Ausführungsbeispiels

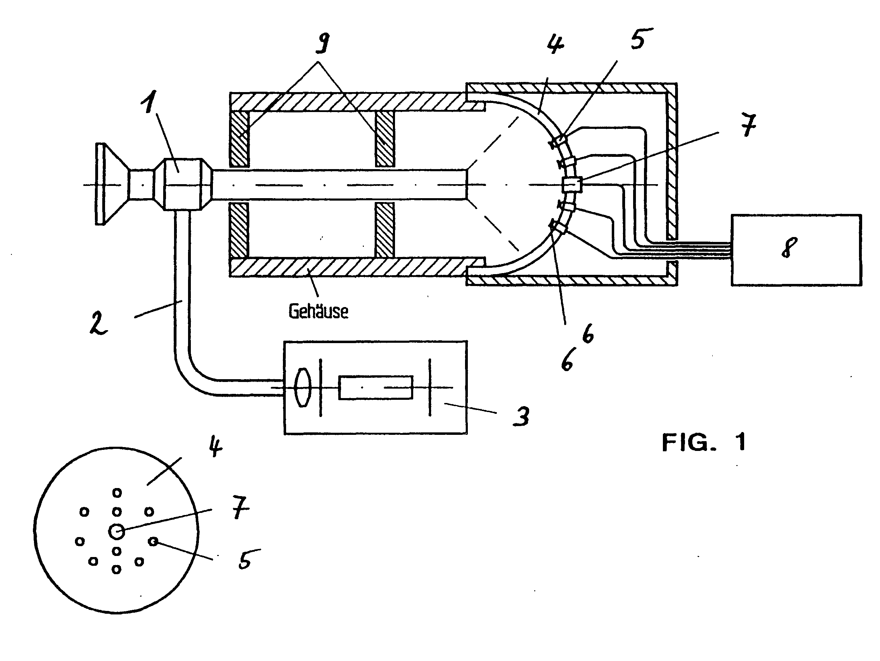

Figur 1 zeigt einen Querschnitt durch eine erfindungsgemäße Vorrichtung zur Prüfung und/oder Justierung von PDD- oder PDT-Systemen bzw. zur Schulung an derartigen Systemen. Bei dem gezeigten Ausführungsbeispiel ist die bildgebende Einheit die das von dem angeregten Gewebebereich kommende Licht abbildet, ohne Beschränkung des allgemeinen Erfindungsgedankens ein starres Endoskop 1. Das Endoskop 1 weist in bekannter Weise ein nichtdargestelltes Objektiv, ein Relaislinsen-System und ein Okular auf, mit dem das von dem am distalen Ende angeordneten Objektiv erzeugte Bild, das das Relais-Linsensystem zum proximalen Ende weiterleitet, betrachtet werden kann. Alternativ zu einer visuellen Betrachtung oder bei Verwendung eines Strahlteilers zusätzlich zu einer visuellen Betrachtung kann an das Okular ebenfalls in bekannter Weise eine Videokamera angeflanscht werden. Ferner weist das Endoskop 1 einen ebenfalls nicht dargestellten Beleuchtungslichtleiter mit einem Lichtleiteranschluß auf, der durch ein Lichtleitkabel 2 mit einem PDD- bzw. PDT-Lichtsystem 3 verbunden ist. Das Lichtsystem 3 weist zumindest ein Beleuchtungssystem, dessen Licht wenigstens zur Anregung des Fluoreszenzstoffes dient und bevorzugt ein Lichtsystem zur Umgebungsbeleuchtung auf, dessen Licht nicht zur Anregung dient. Das Lichtsystem 3 kann mehrere koherente oder inkoherente Lichtquellen oder eine breitbandige Lichtquelle aufweisen, die sowohl zur Anregung des Fluoreszenzfarbstoffes als auch zur Umgebungsbeleuchtung dient. Eine derartige breitbandige Lichtquelle wird beispielsweise von der Karl Storz GmbH & Co., Tuttlingen, Deutschland, hergestellt. Die erfindungsgemäße Vorrichtung weist ein Target 4 auf, das das auf sie auftreffende Licht des Lichtsystems 3 reflektiert und dessen Reflextionseigenschaften den Reflexionseigenschaften des zu untersuchendes Gewebes angepaßt sind. In dem Target 4 sind eine Reihe von Lichtquellen 5 vorgesehen, bei denen es sich um Leuchtdioden, Miniaturlämpchen oder die Lichtaustrittsflächen von Lichtleitfasern handeln kann. Zur Anpassung des Emissionsspektrums der Lichtquellen an das Emissionsspektrum des Fluoreszenzmarkers sind den Lichtquellen Filter 6 vorgeschaltet, die eine geeignete Spektrums-Anpassung ausführen. Ferner ist in dem Target 4 ein Photoelement 7 vorgesehen, das die Stärke des Beleuchtungslichtes insbesondere in dem Wellenlängenbereich erfaßt, in dem der jeweilige Photosensibilisator angeregt wird. Das Ausgangssignal des Photoelements 7 ist an eine Steuereinheit 8 angelegt, die die Lichtstärke der Lichtquellen 5 steuert. Zusätzlich kann die Steuereinheit 8 die Lichtquellen 5 derart selektiv ansteuern, daß sie Licht in Form vorgebbarer Muster emitieren. Das Teilbild links unten in Figur 1 zeigt zur Verdeutlichung der Lage der Lichtquellen 5 und des Photoelements 7 eine Aufsicht auf das Target 4.

Ferner weist die erfindungsgemäße Vorrichtung Spannelemente 9 auf, in die das Endoskop 1 eingesetzt werden kann und die das Endoskop halten. Die Spannelemente 9 sind so ausgebildet, daß sie zusammen mit dem Target 4 einen Hohlraum bilden, so daß das Endoskop unter "Praxisbedingungen" getestet werden kann bzw. die Schulung an dem PDD- bzw. PDT-System unter Praxisbedingungen erfolgen kann.

Im folgenden soll die Funktionsweise der erfindungsgemäßen Vorrichtung beschrieben werden:

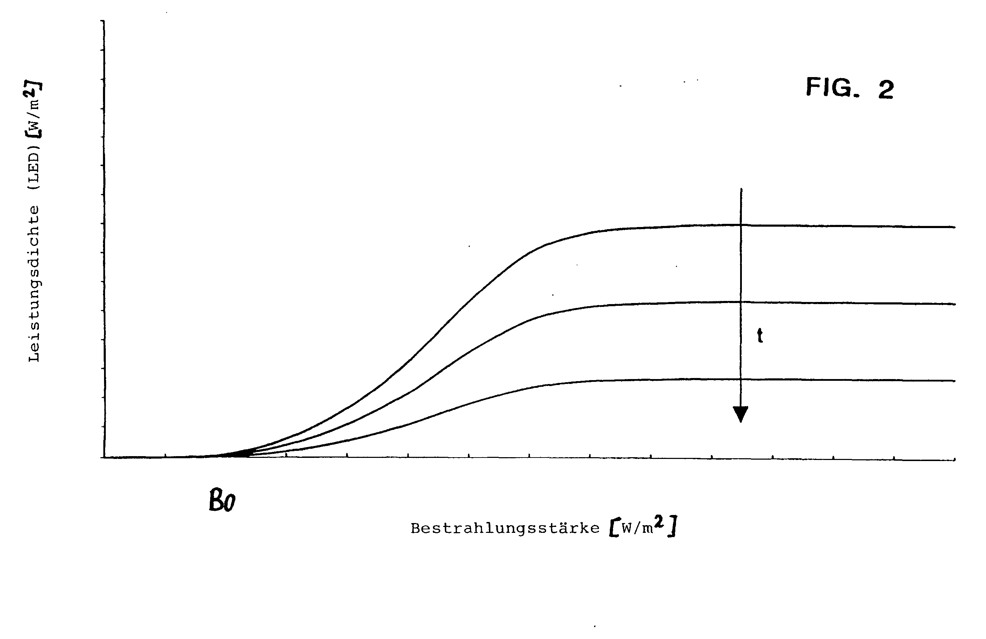

Das Photoelement 7 erfaßt die mittlere Beleuchtungsstärke (B) des Anregungslichtes. Die Steuereinheit 8, an der das Ausgangssignal des Photoelements 7 anliegt, steuert nun die Lichtquellen 5 derart an, daß ihre Abstrahlleistung (Lab) eine Funktion der Beleuchtungsstärke B des Anregungslichtes ist. Figur 2 zeigt mögliche Funktionsverläufe, durch die der Fluoreszenzeffekt im Gewebe möglichst "naturgetreu" nachgebildet wird:

Aufgetragen ist bei diesem Ausführungsbeispiel die Leistungsdichte der als Lichtquellen 5 verwendeten Leuchtdioden als Funktion der Bestrahlungsstärke. Der Figur ist zu entnehmen, daß die Leuchtdioden 5 Licht erst ab einem bestimmten Schwellwert B0 abstrahlen, wobei sie bei weiterer Steigerung der Bestrahlungsstärke nach einem angenähert linear proportionalen Teil in die "Sättigung" übergehen. Der Wert der Sättigung ist dabei zeitabhängig und nimmt entsprechend dem "Verbrauch des Fluoreszenzfarbstoffes" im Laufe der Zeit ab, so daß eine Dosimetriesimulation möglich ist.

Verwendet man als Fluoreszenzfarbstoff Aminolavulinsäure (ALA) so ist es bevorzugt, wenn das Anregungslicht in einem Wellenlängenbereich um 410 nm ± 25 nm liegt und die Leuchtdioden Licht mit einer Wellenlänge von ca. 635 nm abstrahlen.

## Patentansprüche

1. Vorrichtung zur Prüfung und/oder Justierung eines PDD- oder PDT-Systems und/oder zur Schulung an einem derartigen System, das
- ein Beleuchtungssystem (3), dessen Licht wenigstens zur Anregung eines Fluoreszenzstoffs dient und hierzu auf den zu diagnostizierenden und/oder zu therapierenden Gewebebereich gerichtet werden kann, und
- eine bildgebende Einheit (I), die das von dem angeregten Gewebebereich kommende Licht abbildet,
aufweist,
**dadurch gekennzeichnet, daß** ein Target (4) vorgesehen ist, das das Licht des Beleuchtungssystems (3) reflektiert, und daß das Target (4) wenigstens eine Lichtquelle (5) aufweist, die Licht im Wellenlängenbereich des Fluoreszenzspektrums des jeweiligen Fluoreszenzstoffs abstrahlt.

2. Vorrichtung nach Anspruch 1,
**dadurch gekennzeichnet, daß** das Target (4) mehrere Lichtquellen (5) aufweist.

3. Vorrichtung nach Anspruch 1 oder 2,
**dadurch gekennzeichnet, daß** das Target (4) wenigstens einen Sensor aufweist, der die Stärke des Beleuchtungslichtes erfaßt, und dessen Ausgangssignal an einer Steuereinheit (8) anliegt, die die Stärke des von der oder den Lichtquellen emittierten Lichtes regelt.

4. Vorrichtung nach Anspruch 3,
**dadurch gekennzeichnet, daß** der wenigstens eine Sensor die Stärke des Beleuchtungslichtes in dem Wellenlängenbereich erfaßt, in dem der jeweilige Fluoreszenzstoff angeregt wird.

5. Vorrichtung nach einem der Ansprüche 2 bis 4,
**dadurch gekennzeichnet, daß** die Lichtquellen (5) selektiv derart angesteuert werden, daß sie Licht in Form vorgebbarer Muster emittieren.

6. Vorrichtung nach Anspruch 5,
**dadurch gekennzeichnet, daß** die Größe der lichtemittierenden Fläche der Lichtquellen (5) variiert wird.

7. Vorrichtung nach einem der Ansprüche 2 bis 4,
**dadurch gekennzeichnet, daß** die Lichtquellen verschieden große Abstrahlflächen aufweisen.

8. Vorrichtung nach einem der Ansprüche 1 bis 7,
**dadurch gekennzeichnet, daß** das Beleuchtungssystem (3) des PDD- oder PDT-Systems eine weitere Lichtquelle (5) und/oder eine breitbandige Lichtquelle aufweist, so daß zusätzlich zum Anregungslicht Licht eines weiteren Wellenlängenbereiches eingekoppelt wird, das zur Beleuchtung des angeregten Gewebebereichs dient.

9. Vorrichtung nach Anspruch 8,
**dadurch gekennzeichnet, daß** die bildgebende Einheit des PDD- oder PDT-Systems ein Filtersystem aufweist, das das an dem Gewebebereich bzw. dem Target (4) direkt reflektierte Licht abblockt.

10. Vorrichtung nach Anspruch 9,
**dadurch gekennzeichnet, daß** das Filtersystem (6) den Reintransmissionsgrad Til(λ) des Beleuchtungssystems (3) dem Fluoreszenzanregungsspektrum des jeweiligen Fluoreszenzstoffs und den Reintransmissionsgrad Tib(λ) der bildgebenden Einheit dem Fluoreszenzspektrum des jeweiligen Fluoreszenzstoffes derart anpaßt, daß sich die beiden Reintransmissionsgrade nur in einem vergleichsweise kleinen Wellenlängenbereich nennenswert überlappen.

11. Vorrichtung nach einem der Ansprüche 8 bis 10,
**dadurch gekennzeichnet, daß** die Intensität und/oder die Wellenlängen-Erstreckung des lediglich zur Beleuchtung dienenden Lichts einstellbar ist.

12. Vorrichtung nach einem der Ansprüche 9 bis 11,
**dadurch gekennzeichnet, daß** die Transmissionskurve des Filtersystems einstellbar ist.

13. Vorrichtung nach einem der Ansprüche 9 bis 12,
**dadurch gekennzeichnet, daß** das Filtersystem der bildgebenden Einheit umschaltbare Filter (6) aufweist.

14. Vorrichtung nach Anspruch 13,
**dadurch gekennzeichnet, daß** die bildgebende Einheit eine Videokamera aufweist, und daß die Filterumschaltung mit einer Frequenz erfolgt, die ein 1/n-faches (n=1..200) der Videofrequenz ist.

15. Vorrichtung nach einem der Ansprüche 1 bis 14,
**dadurch gekennzeichnet, daß** das Target (4) das Licht des Beleuchtungssystems (3) zumindest in dem Teil des Spektrums, der zur Anregung des Fluoreszenzstoffs dient, angenähert wie das Körpergewebe reflektiert, auf das das Licht des Beleuchtungssystems (3) gerichtet werden soll.

16. Vorrichtung nach Anspruch 15,
**dadurch gekennzeichnet, daß** das Target (4) eine Oberflächenstruktur aufweist, die insbesondere bezüglich Farbe und Rauheit in etwa der des zu simulierenden Gewebes entspricht.

17. Vorrichtung nach einem der Ansprüche 1 bis 16 in Verbindung mit Anspruch 8,
**dadurch gekennzeichnet, daß** das Target (4) in Einfalls-richtung des Beleuchtungslichtes vor der Fläche, in der sich die Lichtquelle(n) befindet(n), eine wellenlängen-selektiv reflektierende Fläche oder ein Filterelement (6) aufweist.

18. Vorrichtung nach einem der Ansprüche 1 bis 17 in Verbindung mit Anspruch 8,
**dadurch gekennzeichnet, daß** das PDD- bzw. PDT-System ein Endoskop (1) oder ein Operationsmikroskop aufweist, in das das Beleuchtungslicht eingekoppelt wird, und dessen Abbildungssystem Teil der bildgebenden Einheit ist.

19. Vorrichtung nach Anspruch 18,
**dadurch gekennzeichnet, daß** die Vorrichtung einen Hohlraum aufweist, in den zur Prüfung und/oder zur Schulung das distale Ende des jeweils verwendeten Endoskops (1) bzw. des Mikroskop-Objektivs eingesetzt wird.

20. Vorrichtung nach Anspruch 19,
**dadurch gekennzeichnet, daß** in den Hohlraum eine Sterilfolie eingesetzt werden kann, die das jeweils eingeführte sterile Teil vor Kontamination schützt.

21. Vorrichtung nach einem der Ansprüche 1 bis 20,
**dadurch gekennzeichnet, daß** das Target (4) eine Krümmung entsprechend der Objektfeldkrümmung der bildgebenden Einheit aufweist.

22. Vorrichtung nach einem der Ansprüche 1 bis 21,
**dadurch gekennzeichnet, daß** die wenigstens eine Lichtquelle (5) eine Leuchtdiode ist.

23. Vorrichtung nach einem der Ansprüche 1 bis 21,
**dadurch gekennzeichnet, daß** die wenigstens eine Lichtquelle (5) ein Miniaturlämpchen ist.

24. Vorrichtung nach einem der Ansprüche 1 bis 21,
**dadurch gekennzeichnet, daß** die wenigstens eine Lichtquelle (5) ein Bildschirm ist.

25. Vorrichtung nach einem der Ansprüche 1 bis 21,
dadurch **gskennzeichnet,** daß die wenigstens eine Lichtquelle (5) die Austrittsfläche eines Lichtleiters oder eines Lichtleiterbündels ist.

26. Vorrichtung nach einem der Ansprüche 1 bis 25,
**dadurch gekennzeichnet, daß** der wenigstens einen Lichtquelle (5) ein Filtersystem vorgeschaltet ist, das das Spektrum der Lichtquelle (5) dem Fluoreszenzspektrum des jeweiligen Fluoreszenzstoffs anpaßt.

27. Vorrichtung nach einem der Ansprüche 1 bis 26,
**dadurch gekennzeichnet, daß** eine Steuer- und Auswerteeinheit (8) vorgesehen ist, die nach einem vorgebbaren Programmablauf die einzelnen Funktionen steuert.

28. Vorrichtung nach Anspruch 27,
**dadurch gekennzeichnet, daß** an der Steuer- und Auswerteeinheit (8) das Ausgangssignal eines Bildsensors der bildgebenden Einheit angelegt ist.

## Claims

1. Device for testing and/or setting a PDD or PDT system, and/or for the training on such a system, comprising
- an illuminating system (3) whose light serves at least to stimulate a fluorescent substance and can be directed, to this end, on the tissue area to be diagnose and/or treated, and
- a lens unit (1, 4, 5) that images the object field into an imaging plane orthogonal on the longitudinal axis (12), and
- an imaging unit (1) that images the light arriving from the stimulated tissue area,
**characterised in that** a target (4) is provided that reflects the light of the illuminating system (3), and
in that said target (4) comprises at least one light source (5) emitting light within the wavelength range of the fluorescence spectrum of said respective fluorescent substance.

2. Device according to Claim 1,
**characterised in that** said target (4) comprises several light sources.

3. Device according to Claim 1 or 2,
**characterised in that** said target (4) comprises at least one sensor detecting the intensity of the illuminating light, whose output signal is applied to a controller (9) that controls the intensity of the light emitted by said light source or sources.

4. Device according to Claim 3,
**characterised in that** said at least one sensor detects the intensity of the illuminating light within the wavelength range within which said fluorescent substance is stimulated.

5. Device according to any of the Claims 2 to 4,
**characterised in that** said light sources (5) are selectively controlled in a way that they emit light in the form of definable patterns.

6. Device according to Claim 5,
**characterised in that** the size of the light-emitting surface of said light sources (5) is varied.

7. Device according to any of the Claims 2 to 4,
**characterised in that** said light sources comprise emitting surfaces of different sizes.,

8. Device according to any of the Claims 1 to 7,
**characterised in that** said illuminating system (3) of said PDD or PDT system comprises another light source (5) and/or a wide-band light source so as to introduce light within a wavelength range additional to said stimulating light, that serves to illuminate the stimulated tissue area.

9. Device according to Claim 8,
**characterised in that** said imaging unit of the PDD or PDT system comprises a filter system that suppresses the light directly reflected at said tissue area or at said target (4).

10. Device according to Claim 9,
**characterised in that** said filter system (6) adapts the internal transmission factor Til(λ) of said illuminating system (3) to the spectrum of fluorescence spectrum of the respective fluorescent substance and the internal transmission factor Tib(λ) of said imaging unit to the fluorescence spectrum of said respective fluorescent substance in a way that the two internal transmission factors overlap each other only within a comparatively small wavelength range.

11. Device according to any of the Claims 8 to 10,
**characterised in that** the intensity and/or extent of the wavelengths of the light is controllable that serves only for illumination.

12. Device according to any of the Claims 9 to 11,
**characterised in that** the characteristic of transmission of the filter system is controllable.

13. Device according to any of the Claims 9 to 12,
**characterised in that** said filter system of said imaging unit comprises switchable filters (6).

14. Device according to Claim 13,
**characterised in that** said imaging unit comprises a video camera, and
in that the filters are switched at a frequency corresponding to 1/n times (n = 1...200) the video frequency.

15. Device according to any of the Claims 1 to 14,
**characterised in that** said target (4) reflects the light of said illuminating system (3) at least in that part of the spectrum that serves to stimulate the fluorescent substance in a manner approximating that of the body tissue, onto which the light of said illuminating system (3) is to be directed.

16. Device according to Claim 15,
**characterised in that** said target (4) has a surface structure that corresponds approximately to the structure of the tissue to be simulated, particularly as far as the colour and the roughness are concerned.

17. Device according to any of the Claims 1 to 16 in combination with Claim 8,
**characterised in that** said target (4) comprises a surface selectively reflecting in terms of wavelengths, or a filter element (6), which are arranged upstream of the surface - when seen in the direction of incidence of the illuminating light - in which the illuminating source(s) is/are located.

18. Device according to any of the Claims 1 to 17 in combination with Claim 8,
**characterised in that** said PDD or PDT system comprises an endoscope (1) or a surgical microscope wherein the illuminating light is introduced, and whose imaging system constitutes one part of said imaging unit.

19. Device according to Claim 18,
**characterised in that** the device comprises a cavity into which the distal end of the respectively employed endoscope (1) or the lens of a microscope is inserted for testing and/or training.

20. Device according to Claims 19,
**characterised in that** a sterile sheet can be inserted into said cavity, that protects the respectively introduced sterile element from contamination.

21. Device according to any of the Claims 1 to 20,
**characterised in that** said target (4) presents a curvature in correspondence with the object field of said imaging unit.

22. Device according to any of the Claims 1 to 21,
**characterised in that** said at least one light source (5) is a light-emitting diode.

23. Device according to any of the Claims 1 to 21,
**characterised in that** said at least one light source (5) is a miniature lamp.

24. Device according to any of the Claims 1 to 21,
**characterised in that** said at least one light source (5) is a screen.

25. Device according to any of the Claims 1 to 21,
**characterised in that** said at least one light source (5) is the exit surface of a Fibre optical light guide or of a bundle of Fibre optical light guides.

26. Device according to any of the Claims 1 to 25,
**characterised in that** a filter system is connected upstream of said at least one light source (5), that adapts the spectrum of said light source (5) to the fluorescence spectrum of said respective fluorescent substance.

27. Device according to any of the Claims 1 to 26,
**characterised in that** a controller and analyser unit (8) is provided that controls the individual functions en correspondence with a predeterminable program sequence.

28. Device according to Claim 28,
**characterised in that** the output signal of a video detector of said imaging unit is applied to said controller and analyser unit (8).

## Revendications

1. Dispositif de contrôle et/ou de réglage d'un système PDD ou PDT, et/ou pour la formation sur un tel système, qui comprend
- un système d'éclairage (3), dont la lumière sert au moins à stimuler une substance fluorescente et se peut diriger à cette fin sur la zone de tissu à diagnostiquer et/ou à traiter, et
- une unité lentilles (1, 4, 5) qui crée l'image du champ d'objet dans un plan d'image orthogonal sur l'axe longitudinal (12), et
- une unité d'établissement d'images (1), qui produit l'image de la lumière qui vient de la zone de tissu stimulé,
**caractérisé en ce qu'**une cible (4) est disposé, qui réfléchit la lumière du système d'éclairage (3), et
en ce que ladite cible (4) comprend au moins une source lumineuse (5), qui émet de la lumière au-dedans de la gamme de longueurs d'onde du spectre de fluorescence de ladite substance fluorescente respective.

2. Dispositif selon la revendication 1,
**caractérisé en ce que** ladite cible (4) comprend plusieurs sources lumineuses.

3. Dispositif selon la revendication 1 ou 2,
**caractérisé en ce que** ladite cible (4) comprend au moins un détecteur, qui détecte l'intensité de la lumière d'éclairage, et dont le signal de sortie est appliqué à une unité de commande (9), qui règle l'intensité de la lumière émise par ladite ou lesdites sources lumineuses.

4. Dispositif selon la revendication 3,
**caractérisé en ce que** ledit au moins un détecteur détecte l'intensité de la lumière d'éclairage au-dedans de la gamme de longueurs d'onde, dans laquelle ladite substance fluorescente est stimulée.

5. Dispositif selon une quelconque des revendications 2 à 4,
**caractérisé en ce que** lesdites sources lumineuses (5) sont sélectivement commandées de façon, qu'elles émettent de la lumière sous forme de dessins ou structures définissables.

6. Dispositif selon la revendication 5,
**caractérisé en ce que** les dimensions de la surface émettrice desdites sources lumineuses (5) sont variées.

7. Dispositif selon une quelconque des revendications 2 à 4,
**caractérisé en ce que** lesdites sources lumineuses comprennent des surfaces émettrices de dimensions différentes.

8. Dispositif selon une quelconque des revendications 1 à 7,
**caractérisé en ce que** ledit système d'éclairage (3) dudit système PDD ou PDT comprend une autre source lumineuse (5) et/ou une source lumineuse à large bande, de façon à introduire de la lumière dans une gamme de longueurs d'onde supplémentaire au plus de ladite lumière stimulante, qui sert à éclairer la zone de tissu stimulée.

9. Dispositif selon la revendication 8,
**caractérisé en ce que** ladite unité d'établissement d'images du système PDD ou PDT comprend un système de filtres, qui bloque la lumière directement réfléchie à ladite zone de tissu ou à ladite cible (4).

10. Dispositif selon la revendication 9,
**caractérisé en ce que** ledit système de filtres (6) adapte le facteur de transmission Til(λ) dudit système d'éclairage (3) au spectre de stimulation de fluorescence de la substance fluorescente respective et le facteur de transmission Tib(λ) de ladite unité d'établissement d'images au spectre de fluorescence de ladite substance fluorescente respective, d'une manière que les deux facteurs de transmission se chevauchent seulement au-dedans une relativement petite gamme de longueurs d'onde.

11. Dispositif selon une quelconque des revendications 8 à 10,
**caractérisé en ce que** l'intensité et/ou l'étendue de longueurs d'onde de la lumière qui sert seulement à l'éclairage est réglable.

12. Dispositif selon une quelconque des revendications 9 à 11,
**caractérisé en ce que** la courbe de transmission du système de filtres est réglable.

13. Dispositif selon une quelconque des revendications 9 à 12,
**caractérisé en ce que** ledit système de filtres de ladite unité d'établissement d'images comprend des filtres échangeables (6).

14. Dispositif selon la revendication 13,
**caractérisé en ce que** ladite unité d'établissement d'images comprend une caméra vidéo, et
en ce que l'échange de filtres se fait à une fréquence, qui correspond à 1/n fois (n = 1...200) de la fréquence vidéo.

15. Dispositif selon une quelconque des revendications 1 à 14,
**caractérisé en ce que** ladite cible (4) réfléchit la lumière dudit système d'éclairage (3) au moins dans cette partie du spectre, qui sert à stimuler la substance fluorescente, de façon approchée au tissu corporel, sur lequel on doit diriger la lumière dudit système d'éclairage (3).

16. Dispositif selon la revendication 15,
**caractérisé en ce que** ladite cible (4) présente une structure superficielle, qui correspond environ à la structure du tissu à simuler, en particulier en ce qui concerne la couleur et la rugosité.

17. Dispositif selon une quelconque des revendications 1 à 16 en combinaison avec la revendication 8,
**caractérisé en ce que** ladite cible (4) comprend une surface réfléchissante, sélective en ce qui concerne les longueurs d'onde ou un élément filtre (6), qui sont disposés, vue en sens d'incidence de la lumière d'éclairage, an amont de la face dans laquelle se trouve(nt) la/les source(s) lumineuse(s).

18. Dispositif selon une quelconque des revendications 1 à 17 en combinaison avec la revendication 8,
**caractérisé en ce que** ledit système PDD ou PDT comprend un endoscope (1) ou un microscope chirurgical, dans lequel la lumière d'éclairage est introduite, et dont le système d'image constitue une partie de ladite unité d'établissement d'images.

19. Dispositif selon la revendication 18,
**caractérisé en ce que** le dispositif comprend une cavité, dans laquelle l'extrémité distale de l'endoscope respectif utilisé (1) ou de l'objectif d'un microscope est inséré pour le contrôle et/ou la formation.

20. Dispositif selon la revendications 19,
**caractérisé en ce qu'**on peut insérer une feuille stérile dans ladite cavité, qui protège l'élément stérile respectif introduit contre une contamination.

21. Dispositif selon une quelconque des revendications 1 à 20,
**caractérisé en ce que** ladite cible (4) présente une courbure en correspondance avec la courbure du champ d'objet de ladite unité d'établissement d'images.

22. Dispositif selon une quelconque des revendications 1 à 21,
**caractérisé en ce que** ladite au moins une source lumineuse (5) est une diode électroluminescente DEL.

23. Dispositif selon une quelconque des revendications 1 à 21,
**caractérisé en ce que** ladite au moins une source lumineuse (5) est une lampe en miniature.

24. Dispositif selon une quelconque des revendications 1 à 21,
**caractérisé en ce que** ladite au moins une source lumineuse (5) est un écran.

25. Dispositif selon une quelconque des revendications 1 à 21,
**caractérisé en ce que** ladite au moins une source lumineuse (5) est la surface de sortie d'un guide de lumière ou d'un nappe de guides de lumière.

26. Dispositif selon une quelconque des revendications 1 à 25,
**caractérisé en ce qu'**un système de filtres est relié en amont de ladite au moins une source lumineuse (5), qui adapte le spectre de ladite source lumineuse (5) au spectre de fluorescence de la substance fluorescente respective.

27. Dispositif selon une quelconque des revendications 1 à 26,
**caractérisé en ce qu'**une unité de commande et d'analyse (8) est disposée, qui commande les fonctions individuelles en correspondance avec une suite d'étapes de programme déterminable.

28. Dispositif selon la revendication 28,
**caractérisé en ce que** le signal de sortie d'un détecteur vidéo de ladite unité d'établissement d'images est appliqué à ladite unité de commande et d'analyse (8).
